# EUROPEAN PATENT APPLICATION

(11) **EP 3 715 465 A1**
(43) Date of publication of application: **30.09.2020**
(21) Application number: 19166333.5
(22) Date of filing: 29.03.2019
(51) Int. Cl.: C12P 19/02, C12P 19/24, C12N 9/90

(54) **TEMPERATURE OPTIMIZED L-ARABINOSE ISOMERASE MUTANTS**

(71) Applicant: Clariant Produkte (Deutschland) GmbH, 81925 München (DE)
(72) Inventor: FARWICK, Alexander, 81373 München (DE); CLAREN, Jörg, 82131 Stockdorf (DE); DIETZ, Heiko, 27638 Wurster Nordseeküste (DE); JOCHENS, Helge, 82065 Baierbrunn (DE); MERTEL, Magdalena, 85247 Schwabhausen (DE)
(74) Representative: Graser, Konstanze

(57) **Abstract**

The present invention relates to a temperature optimized L-arabinose isomerase with a high catalytic activity within feedstocks comprising a high concentration of divalent metal ions, a nucleic acid sequence encoding the inventive L-arabinose isomerase, a vector comprising the nucleic acid sequence encoding the inventive L-arabinose isomerase, a composition containing the inventive L-arabinose isomerase, a yeast cell comprising the inventive L-arabinose isomerase, the use of the inventive L-arabinose isomerase, the composition or the yeast cell for the fermentation of feedstock with a high content of divalent metal ions.

SEQUENCE LISTING

<110> Clariant Produkte (Deutschland) GmbH

<120> Temperature optimized L-arabinose isomerase mutants

<130> 2019DE606

<160> 17

<170> BiSSAP 1.3.6

<210> 1
<211> 1407
<212> DNA
<213> Artificial Sequence

<220>
<223> modified L-arabinose isomerase, codon-optimized

<400> 1
atgttgaaga ccccagatta cgaattctgg ttcgctgtcg gttcccaacc attgtacggt 60

ccagaagctt tggaacaagt cgaaaaggat ggtagaaagt tggtcgatgg tttgaacaag 120

aaggacttgc catacaaggt cgttttcaag ttggtcgcta ccactgctga ttccatcacc 180

aagttcatga aggatgctaa ctacaacgat aaggtcgctg gtgtcatgac ctggatgcat 240

accttctccc cagctaagaa ctggatcaga ggtacccaat tgttacaaaa gccattgtta 300

catttggcta cccaaatgtt ggatcatatc ccattcgata ccatcgactt cgattacatg 360

aacttgaacc aatccgctca tggtgataga gaatacgatt acatcaacgc tagattgggt 420

gtcccagaaa agatcgtcta cggttggtgg aacgatccag aagtccaaga agaaatcgct 480

gattggcaaa aggtcgctat cgcctacgat atgtccttca agatcaagat tgctagattc 540

ggtgatacca tgagagatgt cgctgttacc gaaggtgata aggtcgctgc ccaaatcaag 600

ttgggttgga ccgtcgatta ctggccagtc gctgaattgg tcgctgccgt caacgctgtc 660

tccgaagctg aaatcgatga acaatacaag agattggaag ataactacga cattgaccca 720

aaggcttcta tggattccgt cagataccaa ttgagagaat acttgggtat caagcatttc 780

ttggatgaaa agggttacga tgctttcacc actaacttcc aagatttgga aggtttggaa 840

caattgccag gtttggctgt ccaattgtta atgatcgatg gttacggttt cggtgctgaa 900

ggtgatttca agtccgctgg tttgtctaga ttgttaaaga tcgctgccga aaacaaggaa 960

accgctttca tggaagatta caccttggat ttgagagctg gtcatcaagc tatcatgggt 1020

tcccatatgt tggaagtcga tccaaccttg gcttccgata agccaagagt cgaagttcat 1080

ccattggata tcggtgacaa ggctgatcca gctagattgg tcttcaccgg ttccgaaggt 1140

gaaggtatcg atgtcacctt gtcctacttc gatgacggtt acaagttcat cgcttacgat 1200

gtcgactgtg ctaagccaga aaaggaaatg ccaaacttgc cagtcgctaa gcaaatgtgg 1260

accccaaagt gtggtttgaa gaaaggtgct accgcctgga tgcataacgg tggtggtcat    1320

cacaccgtct tgtccatgaa cttgaccatg gatcaaatgg ctaccttggc taacttgttc    1380

ggtatcgaat tgattgatat caagtga                                        1407

<210> 2
<211> 468
<212> PRT
<213> Artificial Sequence

<220>
<223> modified L-arabinose isomerase

<400> 2
Met Leu Lys Thr Pro Asp Tyr Glu Phe Trp Phe Ala Val Gly Ser Gln
1               5                   10                  15
Pro Leu Tyr Gly Pro Glu Ala Leu Glu Gln Val Glu Lys Asp Gly Arg
            20                  25                  30
Lys Leu Val Asp Gly Leu Asn Lys Lys Asp Leu Pro Tyr Lys Val Val
        35                  40                  45
Phe Lys Leu Val Ala Thr Thr Ala Asp Ser Ile Thr Lys Phe Met Lys
    50                  55                  60
Asp Ala Asn Tyr Asn Asp Lys Val Ala Gly Val Met Thr Trp Met His
65                  70                  75                  80
Thr Phe Ser Pro Ala Lys Asn Trp Ile Arg Gly Thr Gln Leu Leu Gln
                85                  90                  95
Lys Pro Leu Leu His Leu Ala Thr Gln Met Leu Asp His Ile Pro Phe
            100                 105                 110
Asp Thr Ile Asp Phe Asp Tyr Met Asn Leu Asn Gln Ser Ala His Gly
        115                 120                 125
Asp Arg Glu Tyr Asp Tyr Ile Asn Ala Arg Leu Gly Val Pro Glu Lys
    130                 135                 140
Ile Val Tyr Gly Trp Trp Asn Asp Pro Glu Val Gln Glu Glu Ile Ala
145                 150                 155                 160
Asp Trp Gln Lys Val Ala Ile Ala Tyr Asp Met Ser Phe Lys Ile Lys
                165                 170                 175
Ile Ala Arg Phe Gly Asp Thr Met Arg Asp Val Ala Val Thr Glu Gly
            180                 185                 190
Asp Lys Val Ala Ala Gln Ile Lys Leu Gly Trp Thr Val Asp Tyr Trp
        195                 200                 205
Pro Val Ala Glu Leu Val Ala Ala Val Asn Ala Val Ser Glu Ala Glu
    210                 215                 220
Ile Asp Glu Gln Tyr Lys Arg Leu Glu Asp Asn Tyr Asp Ile Asp Pro
225                 230                 235                 240
Lys Ala Ser Met Asp Ser Val Arg Tyr Gln Leu Arg Glu Tyr Leu Gly
                245                 250                 255
Ile Lys His Phe Leu Asp Glu Lys Gly Tyr Asp Ala Phe Thr Thr Asn
            260                 265                 270
Phe Gln Asp Leu Glu Gly Leu Glu Gln Leu Pro Gly Leu Ala Val Gln
        275                 280                 285
Leu Leu Met Ile Asp Gly Tyr Gly Phe Gly Ala Glu Gly Asp Phe Lys
    290                 295                 300
Ser Ala Gly Leu Ser Arg Leu Leu Lys Ile Ala Ala Glu Asn Lys Glu
305                 310                 315                 320
Thr Ala Phe Met Glu Asp Tyr Thr Leu Asp Leu Arg Ala Gly His Gln
                325                 330                 335
Ala Ile Met Gly Ser His Met Leu Glu Val Asp Pro Thr Leu Ala Ser
            340                 345                 350
Asp Lys Pro Arg Val Glu Val His Pro Leu Asp Ile Gly Asp Lys Ala

355                 360                 365
Asp Pro Ala Arg Leu Val Phe Thr Gly Ser Glu Gly Glu Gly Ile Asp
    370                 375                 380
Val Thr Leu Ser Tyr Phe Asp Asp Gly Tyr Lys Phe Ile Ala Tyr Asp
385                 390                 395                 400
Val Asp Cys Ala Lys Pro Glu Lys Glu Met Pro Asn Leu Pro Val Ala
                405                 410                 415
Lys Gln Met Trp Thr Pro Lys Cys Gly Leu Lys Lys Gly Ala Thr Ala
            420                 425                 430
Trp Met His Asn Gly Gly Gly His His Thr Val Leu Ser Met Asn Leu
        435                 440                 445
Thr Met Asp Gln Met Ala Thr Leu Ala Asn Leu Phe Gly Ile Glu Leu
    450                 455                 460
Ile Asp Ile Lys
465

<210> 3
<211> 1425
<212> DNA
<213> Artificial Sequence

<220>
<223> wildtype L-arabinose isomerase, codon-optimized

<400> 3
   atgttgaaga ccccagatta cgaattctgg ttcgctgtcg gttcccaacc attgtacggt      60
   ccagaagctt tggaacaagt cgaaaaggat ggtagaaagt tggtcgatgg tttgaacgct     120
   tccggtaagt tgccatacaa ggtcgttttc aagttggtcg ctaccactgc tgattccatc     180
   accaagttca tgaaggatgc taactacaac gataaggtcg ctggtgtcat gacctggatg     240
   cataccttct ccccagctaa gaactggatc agaggtaccc aattgttaca aaagccattg     300
   ttacatttgg ctacccaaat gttggatcat atcccattcg ataccatcga cttcgattac     360
   atgaacttga accaatccgc tcatggtgat agagaatacg attacatcaa cgctagattg     420
   ggtgtcccag aaaagatcgt ctacggttgg tggaacgatc cagaagtcca agaagaaatc     480
   gctgattggc aaaaggtcgc tatcgcctac gatatgtcct tcaagatcaa gattgctaga     540
   ttcggtgata ccatgagaga tgtcgctgtt accgaaggtg ataaggtcgc tgcccaaatc     600
   aagttgggtt ggaccgtcga ttactggcca gtcgctgaat tggtcgctgc cgtcaacgct     660
   gtctccgaag ctgaaatcga tgaacaatac aagagattgg aagataacta cgacatggtc     720
   gaaggtgata acgaccatga taagtacgtc cattccgtca gataccaatt gagagaatac     780
   ttgggtatca agcatttctt ggatgaaaag ggttacgatg ctttcaccac taacttccaa     840
   gatttggaag gtttggaaca attgccaggt ttggctgtcc aattgttaat gatcgatggt     900
   tacggtttcg gtgctgaagg tgatttcaag tccgctggtt tgtctagatt gttaaagatc     960
   gctgccgaaa acaaggaaac cgctttcatg gaagattaca ccttggattt gagagctggt    1020
   catcaagcta tcatgggttc ccatatgttg gaagtcgatc caaccttggc ttccgataag    1080
   ccaagagtcg aagttcatcc attggatatc ggtgacaagg ctgatccagc tagattggtc    1140
   ttcaccggtt ccgaaggtga aggtatcgat gtcaccttgt cctacttcga tgacggttac 1200
   aagttcatcg cttacgatgt cgactgtgct aagccagaaa aggaaatgcc aaacttgcca 1260
   gtcgctaagc aaatgtggac cccaaagtgt ggtttgaaga aaggtgctac cgcctggatg 1320
   cataacggtg gtggtcatca caccgtcttg tccatgaact tgaccatgga tcaaatggct 1380
   accttggcta acttgttcgg tatcgaattg attgatatca agtga 1425

<210> 4
<211> 474
<212> PRT
<213> Lactobacillus antri DSM 16041

<220>
<223> wildtype L-arabinose isomerase

<400> 4
Met Leu Lys Thr Pro Asp Tyr Glu Phe Trp Phe Ala Val Gly Ser Gln
1               5                   10                  15
Pro Leu Tyr Gly Pro Glu Ala Leu Glu Gln Val Glu Lys Asp Gly Arg
            20                  25                  30
Lys Leu Val Asp Gly Leu Asn Ala Ser Gly Lys Leu Pro Tyr Lys Val
        35                  40                  45
Val Phe Lys Leu Val Ala Thr Thr Ala Asp Ser Ile Thr Lys Phe Met
    50                  55                  60
Lys Asp Ala Asn Tyr Asn Asp Lys Val Ala Gly Val Met Thr Trp Met
65                  70                  75                  80
His Thr Phe Ser Pro Ala Lys Asn Trp Ile Arg Gly Thr Gln Leu Leu
                85                  90                  95
Gln Lys Pro Leu Leu His Leu Ala Thr Gln Met Leu Asp His Ile Pro
            100                 105                 110
Phe Asp Thr Ile Asp Phe Asp Tyr Met Asn Leu Asn Gln Ser Ala His
        115                 120                 125
Gly Asp Arg Glu Tyr Asp Tyr Ile Asn Ala Arg Leu Gly Val Pro Glu
    130                 135                 140
Lys Ile Val Tyr Gly Trp Trp Asn Asp Pro Glu Val Gln Glu Glu Ile
145                 150                 155                 160
Ala Asp Trp Gln Lys Val Ala Ile Ala Tyr Asp Met Ser Phe Lys Ile
                165                 170                 175
Lys Ile Ala Arg Phe Gly Asp Thr Met Arg Asp Val Ala Val Thr Glu
            180                 185                 190
Gly Asp Lys Val Ala Ala Gln Ile Lys Leu Gly Trp Thr Val Asp Tyr
        195                 200                 205
Trp Pro Val Ala Glu Leu Val Ala Ala Val Asn Ala Val Ser Glu Ala
    210                 215                 220
Glu Ile Asp Glu Gln Tyr Lys Arg Leu Glu Asp Asn Tyr Asp Met Val
225                 230                 235                 240
Glu Gly Asp Asn Asp His Asp Lys Tyr Val His Ser Val Arg Tyr Gln
                245                 250                 255
Leu Arg Glu Tyr Leu Gly Ile Lys His Phe Leu Asp Glu Lys Gly Tyr
            260                 265                 270
Asp Ala Phe Thr Thr Asn Phe Gln Asp Leu Glu Gly Leu Glu Gln Leu
        275                 280                 285
Pro Gly Leu Ala Val Gln Leu Leu Met Ile Asp Gly Tyr Gly Phe Gly
    290                 295                 300
Ala Glu Gly Asp Phe Lys Ser Ala Gly Leu Ser Arg Leu Leu Lys Ile
305                 310                 315                 320
Ala Ala Glu Asn Lys Glu Thr Ala Phe Met Glu Asp Tyr Thr Leu Asp

325                 330                 335
Leu Arg Ala Gly His Gln Ala Ile Met Gly Ser His Met Leu Glu Val
        340                 345                 350
Asp Pro Thr Leu Ala Ser Asp Lys Pro Arg Val Glu Val His Pro Leu
        355                 360                 365
Asp Ile Gly Asp Lys Ala Asp Pro Ala Arg Leu Val Phe Thr Gly Ser
    370                 375                 380
Glu Gly Glu Gly Ile Asp Val Thr Leu Ser Tyr Phe Asp Asp Gly Tyr
385                 390                 395                 400
Lys Phe Ile Ala Tyr Asp Val Asp Cys Ala Lys Pro Glu Lys Glu Met
                405                 410                 415
Pro Asn Leu Pro Val Ala Lys Gln Met Trp Thr Pro Lys Cys Gly Leu
            420                 425                 430
Lys Lys Gly Ala Thr Ala Trp Met His Asn Gly Gly Gly His His Thr
        435                 440                 445
Val Leu Ser Met Asn Leu Thr Met Asp Gln Met Ala Thr Leu Ala Asn
    450                 455                 460
Leu Phe Gly Ile Glu Leu Ile Asp Ile Lys
465                 470

<210> 5
<211> 1446
<212> DNA
<213> Artificial Sequence

<220>
<223> modified L-arabinose isomerase, N-terminal 6xhis-tag, codon
      optimized

<400> 5
atgcatcacc accatcatca cgagaactta tattttcaaa gcttgaagac cccagattac      60

gaattctggt tcgctgtcgg ttcccaacca ttgtacggtc cagaagcttt ggaacaagtc     120

gaaaaggatg gtagaaagtt ggtcgatggt ttgaacaaga aggacttgcc atacaaggtc     180

gttttcaagt tggtcgctac cactgctgat tccatcacca agttcatgaa ggatgctaac     240

tacaacgata aggtcgctgg tgtcatgacc tggatgcata ccttctcccc agctaagaac     300

tggatcagag gtacccaatt gttacaaaag ccattgttac atttggctac ccaaatgttg     360

gatcatatcc cattcgatac catcgacttc gattacatga acttgaacca atccgctcat     420

ggtgatagag aatacgatta catcaacgct agattgggtg tcccagaaaa gatcgtctac     480

ggttggtgga acgatccaga agtccaagaa gaaatcgctg attggcaaaa ggtcgctatc     540

gcctacgata tgtccttcaa gatcaagatt gctagattcg gtgataccat gagagatgtc     600

gctgttaccg aaggtgataa ggtcgctgcc caaatcaagt tgggttggac cgtcgattac     660

tggccagtcg ctgaattggt cgctgccgtc aacgctgtct ccgaagctga aatcgatgaa     720

caatacaaga gattggaaga taactacgac attgacccaa aggcttctat ggattccgtc     780

agataccaat tgagagaata cttgggtatc aagcatttct tggatgaaaa gggttacgat     840

gctttcacca ctaacttcca agatttggaa ggtttggaac aattgccagg tttggctgtc     900

caattgttaa tgatcgatgg ttacggtttc ggtgctgaag gtgatttcaa gtccgctggt     960

ttgtctagat tgttaaagat cgctgccgaa aacaaggaaa ccgctttcat ggaagattac 1020

accttggatt tgagagctgg tcatcaagct atcatgggtt cccatatgtt ggaagtcgat 1080

ccaaccttgg cttccgataa gccaagagtc gaagttcatc cattggatat cggtgacaag 1140

gctgatccag ctagattggt cttcaccggt tccgaaggtg aaggtatcga tgtcaccttg 1200

tcctacttcg atgacggtta caagttcatc gcttacgatg tcgactgtgc taagccagaa 1260

aaggaaatgc caaacttgcc agtcgctaag caaatgtgga ccccaaagtg tggtttgaag 1320

aaaggtgcta ccgcctggat gcataacggt ggtggtcatc acaccgtctt gtccatgaac 1380

ttgaccatgg atcaaatggc taccttggct aacttgttcg gtatcgaatt gattgatatc 1440

aagtga 1446

<210> 6
<211> 481
<212> PRT
<213> Artificial Sequence

<220>
<223> modified L-arabinose isomerase, N-terminal 6xhis-tag

<400> 6
Met His His His His His His Glu Asn Leu Tyr Phe Gln Ser Leu Lys
1               5                   10                  15
Thr Pro Asp Tyr Glu Phe Trp Phe Ala Val Gly Ser Gln Pro Leu Tyr
            20                  25                  30
Gly Pro Glu Ala Leu Glu Gln Val Glu Lys Asp Gly Arg Lys Leu Val
        35                  40                  45
Asp Gly Leu Asn Lys Lys Asp Leu Pro Tyr Lys Val Val Phe Lys Leu
    50                  55                  60
Val Ala Thr Thr Ala Asp Ser Ile Thr Lys Phe Met Lys Asp Ala Asn
65                  70                  75                  80
Tyr Asn Asp Lys Val Ala Gly Val Met Thr Trp Met His Thr Phe Ser
                85                  90                  95
Pro Ala Lys Asn Trp Ile Arg Gly Thr Gln Leu Leu Gln Lys Pro Leu
            100                 105                 110
Leu His Leu Ala Thr Gln Met Leu Asp His Ile Pro Phe Asp Thr Ile
        115                 120                 125
Asp Phe Asp Tyr Met Asn Leu Asn Gln Ser Ala His Gly Asp Arg Glu
    130                 135                 140
Tyr Asp Tyr Ile Asn Ala Arg Leu Gly Val Pro Glu Lys Ile Val Tyr
145                 150                 155                 160
Gly Trp Trp Asn Asp Pro Glu Val Gln Glu Glu Ile Ala Asp Trp Gln
                165                 170                 175
Lys Val Ala Ile Ala Tyr Asp Met Ser Phe Lys Ile Lys Ile Ala Arg
            180                 185                 190
Phe Gly Asp Thr Met Arg Asp Val Ala Val Thr Glu Gly Asp Lys Val
        195                 200                 205
Ala Ala Gln Ile Lys Leu Gly Trp Thr Val Asp Tyr Trp Pro Val Ala
    210                 215                 220
Glu Leu Val Ala Ala Val Asn Ala Val Ser Glu Ala Glu Ile Asp Glu
225                 230                 235                 240
Gln Tyr Lys Arg Leu Glu Asp Asn Tyr Asp Ile Asp Pro Lys Ala Ser
                245                 250                 255
Met Asp Ser Val Arg Tyr Gln Leu Arg Glu Tyr Leu Gly Ile Lys His
            260                 265                 270

Phe Leu Asp Glu Lys Gly Tyr Asp Ala Phe Thr Thr Asn Phe Gln Asp
        275                 280                 285
Leu Glu Gly Leu Glu Gln Leu Pro Gly Leu Ala Val Gln Leu Leu Met
    290                 295                 300
Ile Asp Gly Tyr Gly Phe Gly Ala Glu Gly Asp Phe Lys Ser Ala Gly
305                 310                 315                 320
Leu Ser Arg Leu Leu Lys Ile Ala Ala Glu Asn Lys Glu Thr Ala Phe
                325                 330                 335
Met Glu Asp Tyr Thr Leu Asp Leu Arg Ala Gly His Gln Ala Ile Met
            340                 345                 350
Gly Ser His Met Leu Glu Val Asp Pro Thr Leu Ala Ser Asp Lys Pro
        355                 360                 365
Arg Val Glu Val His Pro Leu Asp Ile Gly Asp Lys Ala Asp Pro Ala
    370                 375                 380
Arg Leu Val Phe Thr Gly Ser Glu Gly Glu Gly Ile Asp Val Thr Leu
385                 390                 395                 400
Ser Tyr Phe Asp Asp Gly Tyr Lys Phe Ile Ala Tyr Asp Val Asp Cys
                405                 410                 415
Ala Lys Pro Glu Lys Glu Met Pro Asn Leu Pro Val Ala Lys Gln Met
            420                 425                 430
Trp Thr Pro Lys Cys Gly Leu Lys Lys Gly Ala Thr Ala Trp Met His
        435                 440                 445
Asn Gly Gly Gly His His Thr Val Leu Ser Met Asn Leu Thr Met Asp
    450                 455                 460
Gln Met Ala Thr Leu Ala Asn Leu Phe Gly Ile Glu Leu Ile Asp Ile
465                 470                 475                 480
Lys

<210> 7
<211> 1464
<212> DNA
<213> Artificial Sequence

<220>
<223> wildtype L-arabinose isomerase, N-terminal 6xhis-tag,
      codon-optimized

<400> 7
atgcatcacc accatcatca cgagaactta tattttcaaa gcttgaagac cccagattac      60

gaattctggt tcgctgtcgg ttcccaacca ttgtacggtc cagaagcttt ggaacaagtc     120

gaaaaggatg gtagaaagtt ggtcgatggt ttgaacgctt ccggtaagtt gccatacaag     180

gtcgttttca agttggtcgc taccactgct gattccatca ccaagttcat gaaggatgct     240

aactacaacg ataaggtcgc tggtgtcatg acctggatgc ataccttctc cccagctaag     300

aactggatca gaggtaccca attgttacaa aagccattgt tacatttggc tacccaaatg     360

ttggatcata tcccattcga taccatcgac ttcgattaca tgaacttgaa ccaatccgct     420

catggtgata gagaatacga ttacatcaac gctagattgg gtgtcccaga aaagatcgtc     480

tacggttggt ggaacgatcc agaagtccaa gaagaaatcg ctgattggca aaaggtcgct     540

atcgcctacg atatgtcctt caagatcaag attgctagat tcggtgatac catgagagat     600

gtcgctgtta ccgaaggtga taaggtcgct gcccaaatca agttgggttg gaccgtcgat     660

tactggccag tcgctgaatt ggtcgctgcc gtcaacgctg tctccgaagc tgaaatcgat     720

gaacaataca agagattgga agataactac gacatggtcg aaggtgataa cgaccatgat 780

aagtacgtcc attccgtcag ataccaattg agagaatact tgggtatcaa gcatttcttg 840

gatgaaaagg gttacgatgc tttcaccact aacttccaag atttggaagg tttggaacaa 900

ttgccaggtt tggctgtcca attgttaatg atcgatggtt acggtttcgg tgctgaaggt 960

gatttcaagt ccgctggttt gtctagattg ttaaagatcg ctgccgaaaa caaggaaacc 1020

gctttcatgg aagattacac cttggatttg agagctggtc atcaagctat catgggttcc 1080

catatgttgg aagtcgatcc aaccttggct tccgataagc caagagtcga agttcatcca 1140

ttggatatcg gtgacaaggc tgatccagct agattggtct tcaccggttc cgaaggtgaa 1200

ggtatcgatg tcaccttgtc ctacttcgat gacggttaca agttcatcgc ttacgatgtc 1260

gactgtgcta agccagaaaa ggaaatgcca aacttgccag tcgctaagca aatgtggacc 1320

ccaaagtgtg gtttgaagaa aggtgctacc gcctggatgc ataacggtgg tggtcatcac 1380

accgtcttgt ccatgaactt gaccatggat caaatggcta ccttggctaa cttgttcggt 1440

atcgaattga ttgatatcaa gtga 1464

<210> 8
<211> 487
<212> PRT
<213> Artificial Sequence

<220>
<223> wildtype L-arabinose isomerase, N-terminal 6xhis-tag

<400> 8
Met His His His His His His Glu Asn Leu Tyr Phe Gln Ser Leu Lys
1               5                   10                  15
Thr Pro Asp Tyr Glu Phe Trp Phe Ala Val Gly Ser Gln Pro Leu Tyr
            20                  25                  30
Gly Pro Glu Ala Leu Glu Gln Val Glu Lys Asp Gly Arg Lys Leu Val
        35                  40                  45
Asp Gly Leu Asn Ala Ser Gly Lys Leu Pro Tyr Lys Val Val Phe Lys
    50                  55                  60
Leu Val Ala Thr Thr Ala Asp Ser Ile Thr Lys Phe Met Lys Asp Ala
65                  70                  75                  80
Asn Tyr Asn Asp Lys Val Ala Gly Val Met Thr Trp Met His Thr Phe
                85                  90                  95
Ser Pro Ala Lys Asn Trp Ile Arg Gly Thr Gln Leu Leu Gln Lys Pro
            100                 105                 110
Leu Leu His Leu Ala Thr Gln Met Leu Asp His Ile Pro Phe Asp Thr
        115                 120                 125
Ile Asp Phe Asp Tyr Met Asn Leu Asn Gln Ser Ala His Gly Asp Arg
    130                 135                 140
Glu Tyr Asp Tyr Ile Asn Ala Arg Leu Gly Val Pro Glu Lys Ile Val
145                 150                 155                 160
Tyr Gly Trp Trp Asn Asp Pro Glu Val Gln Glu Glu Ile Ala Asp Trp
                165                 170                 175
Gln Lys Val Ala Ile Ala Tyr Asp Met Ser Phe Lys Ile Lys Ile Ala
            180                 185                 190
Arg Phe Gly Asp Thr Met Arg Asp Val Ala Val Thr Glu Gly Asp Lys

195                 200                 205
Val Ala Ala Gln Ile Lys Leu Gly Trp Thr Val Asp Tyr Trp Pro Val
    210                 215                 220
Ala Glu Leu Val Ala Ala Val Asn Ala Val Ser Glu Ala Glu Ile Asp
225                 230                 235                 240
Glu Gln Tyr Lys Arg Leu Glu Asp Asn Tyr Asp Met Val Glu Gly Asp
                245                 250                 255
Asn Asp His Asp Lys Tyr Val His Ser Val Arg Tyr Gln Leu Arg Glu
            260                 265                 270
Tyr Leu Gly Ile Lys His Phe Leu Asp Glu Lys Gly Tyr Asp Ala Phe
        275                 280                 285
Thr Thr Asn Phe Gln Asp Leu Glu Gly Leu Glu Gln Leu Pro Gly Leu
    290                 295                 300
Ala Val Gln Leu Leu Met Ile Asp Gly Tyr Gly Phe Gly Ala Glu Gly
305                 310                 315                 320
Asp Phe Lys Ser Ala Gly Leu Ser Arg Leu Leu Lys Ile Ala Ala Glu
                325                 330                 335
Asn Lys Glu Thr Ala Phe Met Glu Asp Tyr Thr Leu Asp Leu Arg Ala
            340                 345                 350
Gly His Gln Ala Ile Met Gly Ser His Met Leu Glu Val Asp Pro Thr
        355                 360                 365
Leu Ala Ser Asp Lys Pro Arg Val Glu Val His Pro Leu Asp Ile Gly
    370                 375                 380
Asp Lys Ala Asp Pro Ala Arg Leu Val Phe Thr Gly Ser Glu Gly Glu
385                 390                 395                 400
Gly Ile Asp Val Thr Leu Ser Tyr Phe Asp Asp Gly Tyr Lys Phe Ile
                405                 410                 415
Ala Tyr Asp Val Asp Cys Ala Lys Pro Glu Lys Glu Met Pro Asn Leu
            420                 425                 430
Pro Val Ala Lys Gln Met Trp Thr Pro Lys Cys Gly Leu Lys Lys Gly
        435                 440                 445
Ala Thr Ala Trp Met His Asn Gly Gly Gly His His Thr Val Leu Ser
    450                 455                 460
Met Asn Leu Thr Met Asp Gln Met Ala Thr Leu Ala Asn Leu Phe Gly
465                 470                 475                 480
Ile Glu Leu Ile Asp Ile Lys
                485

<210> 9
<211> 1407
<212> DNA
<213> Artificial Sequence

<220>
<223> modified L-arabinose isomerase, codon optimized

<400> 9
atgttgaaga ctccagacta cgaattctgg ttcgctgttg gttctcaacc attgtacggt      60

ccagaagctt tggaacaagt tgaaaaggac ggtagaaagt tggttgacgg tttgaacaag     120

aaggacttgc catacaaggt tgtcttcaag ttggttgcta ctaccgctga ctctattact     180

aagttcatga aggacgctaa ctacaacgac aaggttgctg gtgttatgac ttggatgcac     240

actttctctc cagctaagaa ctggattaga ggtactcaat tgttgcaaaa gccattgttg     300

cacttggcta ctcaaatgtt ggaccacatt ccattcgaca ctattgattt cgactacatg     360

aacttgaacc aatctgctca cggtgacaga gaatacgact acattaacgc tagattgggt     420

gttccagaaa agattgttta cggttggtgg aacgacccag aagttcaaga agaaattgct     480

gactggcaaa aggttgctat tgcttacgac atgtctttca agattaagat cgctagattc 540

ggtgacacta tgagagacgt tgctgtcact gaaggtgaca aggttgctgc tcaaattaag 600

ttgggttgga ctgttgacta ctggccagtt gctgaattgg ttgctgctgt taacgctgtt 660

tctgaagctg aaattgacga acaatacaag agattggaag acaactacga tattgaccca 720

aaggcttcta tggactctgt tagataccaa ttgagagaat acttgggtat taagcacttc 780

ttggacgaaa agggttacga cgctttcact accaacttcc aagacttgga aggtttggaa 840

caattgccag gtttggctgt tcaattgttg atgattgacg gttacggttt cggtgctgaa 900

ggtgacttca agtctgctgg tttgtctaga ttgttgaaga ttgctgctga aaacaaggaa 960

actgctttca tggaagacta cactttggac ttgagagctg gtcaccaagc tattatgggt 1020

tctcacatgt tggaagttga cccaactttg gcttctgaca agccaagagt tgaagtccac 1080

ccattggaca ttggtgataa ggctgaccca gctagattgg ttttcactgg ttctgaaggt 1140

gaaggtattg acgttacttt gtcttacttc gacgatggtt acaagttcat tgcttacgac 1200

gttgattgtg ctaagccaga aaaggaaatg ccaaacttgc cagttgctaa gcaaatgtgg 1260

actccaaagt gtggtttgaa gaagggtgct actgcttgga tgcacaacgg tggtggtcac 1320

catactgttt tgtctatgaa cttgactatg gaccaaatgg ctactttggc taacttgttc 1380

ggtattgaat tgatcgacat taagtaa 1407

<210> 10
<211> 1410
<212> DNA
<213> Artificial Sequence

<220>
<223> modified L-arabinose isomerase, codon-optimized

<400> 10
   atgttgaaga ccccagatta cgaattctgg ttcgctgtcg gttcccaacc attgtacggt 60
   ccagaagctt tggaacaagt cgaaaaggat ggtagaaagt tggtcgatgg tttgaacgct 120
   tccggtaagt tgccatacaa ggtcgttttc aagttggtcg ctaccactgc tgattccatc 180
   accaagttca tgaaggatgc taactacaac gataaggtcg ctggtgtcat gacctggatg     240
   cataccttct ccccagctaa gaactggatc agaggtaccc aattgttaca aaagccattg     300
   ttacatttgg ctacccaaat gttggatcat atcccattcg ataccatcga cttcgattac 360
   atgaacttga accaatccgc tcatggtgat agagaatacg attacatcaa cgctagattg 420
   ggtgtcccag aaaagatcgt ctacggttgg tggaacgatc cagaagtcca agaagaaatc 480
   gctgattggc aaaaggtcgc tatcgcctac gatatgtcct tcaagatcaa gattgctaga 540
   ttcggtgata ccatgagaga tgtcgctgtt accgaaggtg ataaggtcgc tgcccaaatc 600
   aagttgggtt ggaccgtcga ttactggcca gtcgctgaat tggtcgctgc cgtcaacgct 660
   gtctccgaag ctgaaatcga tgaacaatac aagagattgg aagataacta cgacattgac 720
   ccaaaggctt ctatggattc cgtcagatac caattgagag aatacttggg tatcaagcat 780
   ttcttggatg aaaagggtta cgatgctttc accactaact tccaagattt ggaaggtttg 840
   gaacaattgc caggtttggc tgtccaattg ttaatgatcg atggttacgg tttcggtgct 900
   gaaggtgatt tcaagtccgc tggtttgtct agattgttaa agatcgctgc cgaaaacaag 960
   gaaaccgctt tcatggaaga ttacaccttg gatttgagag ctggtcatca agctatcatg 1020
   ggttcccata tgttggaagt cgatccaacc ttggcttccg ataagccaag agtcgaagtt 1080
   catccattgg atatcggtga caaggctgat ccagctagat tggtcttcac cggttccgaa 1140
   ggtgaaggta tcgatgtcac cttgtcctac ttcgatgacg gttacaagtt catcgcttac 1200
   gatgtcgact gtgctaagcc agaaaaggaa atgccaaact tgccagtcgc taagcaaatg 1260
   tggaccccaa agtgtggttt gaagaaaggt gctaccgcct ggatgcataa cggtggtggt 1320
   catcacaccg tcttgtccat gaacttgacc atggatcaaa tggctacctt ggctaacttg 1380
   ttcggtatcg aattgattga tatcaagtga 1410

<210> 11
<211> 469
<212> PRT
<213> Artificial Sequence

<220>
<223> modified L-arabinose isomerase

<400> 11
Met Leu Lys Thr Pro Asp Tyr Glu Phe Trp Phe Ala Val Gly Ser Gln
1               5                   10                  15
Pro Leu Tyr Gly Pro Glu Ala Leu Glu Gln Val Glu Lys Asp Gly Arg
            20                  25                  30
Lys Leu Val Asp Gly Leu Asn Ala Ser Gly Lys Leu Pro Tyr Lys Val
        35                  40                  45
Val Phe Lys Leu Val Ala Thr Thr Ala Asp Ser Ile Thr Lys Phe Met
    50                  55                  60
Lys Asp Ala Asn Tyr Asn Asp Lys Val Ala Gly Val Met Thr Trp Met
65                  70                  75                  80
His Thr Phe Ser Pro Ala Lys Asn Trp Ile Arg Gly Thr Gln Leu Leu
                85                  90                  95
Gln Lys Pro Leu Leu His Leu Ala Thr Gln Met Leu Asp His Ile Pro
            100                 105                 110
Phe Asp Thr Ile Asp Phe Asp Tyr Met Asn Leu Asn Gln Ser Ala His
        115                 120                 125
Gly Asp Arg Glu Tyr Asp Tyr Ile Asn Ala Arg Leu Gly Val Pro Glu
    130                 135                 140
Lys Ile Val Tyr Gly Trp Trp Asn Asp Pro Glu Val Gln Glu Glu Ile
145                 150                 155                 160
Ala Asp Trp Gln Lys Val Ala Ile Ala Tyr Asp Met Ser Phe Lys Ile
                165                 170                 175
Lys Ile Ala Arg Phe Gly Asp Thr Met Arg Asp Val Ala Val Thr Glu

180                 185                 190
Gly Asp Lys Val Ala Ala Gln Ile Lys Leu Gly Trp Thr Val Asp Tyr
        195                 200                 205
Trp Pro Val Ala Glu Leu Val Ala Ala Val Asn Ala Val Ser Glu Ala
    210                 215                 220
Glu Ile Asp Glu Gln Tyr Lys Arg Leu Glu Asp Asn Tyr Asp Ile Asp
225                 230                 235                 240
Pro Lys Ala Ser Met Asp Ser Val Arg Tyr Gln Leu Arg Glu Tyr Leu
                245                 250                 255
Gly Ile Lys His Phe Leu Asp Glu Lys Gly Tyr Asp Ala Phe Thr Thr
            260                 265                 270
Asn Phe Gln Asp Leu Glu Gly Leu Glu Gln Leu Pro Gly Leu Ala Val
        275                 280                 285
Gln Leu Leu Met Ile Asp Gly Tyr Gly Phe Gly Ala Glu Gly Asp Phe
    290                 295                 300
Lys Ser Ala Gly Leu Ser Arg Leu Leu Lys Ile Ala Ala Glu Asn Lys
305                 310                 315                 320
Glu Thr Ala Phe Met Glu Asp Tyr Thr Leu Asp Leu Arg Ala Gly His
                325                 330                 335
Gln Ala Ile Met Gly Ser His Met Leu Glu Val Asp Pro Thr Leu Ala
            340                 345                 350
Ser Asp Lys Pro Arg Val Glu Val His Pro Leu Asp Ile Gly Asp Lys
        355                 360                 365
Ala Asp Pro Ala Arg Leu Val Phe Thr Gly Ser Glu Gly Glu Gly Ile
    370                 375                 380
Asp Val Thr Leu Ser Tyr Phe Asp Asp Gly Tyr Lys Phe Ile Ala Tyr
385                 390                 395                 400
Asp Val Asp Cys Ala Lys Pro Glu Lys Glu Met Pro Asn Leu Pro Val
                405                 410                 415
Ala Lys Gln Met Trp Thr Pro Lys Cys Gly Leu Lys Lys Gly Ala Thr
            420                 425                 430
Ala Trp Met His Asn Gly Gly Gly His His Thr Val Leu Ser Met Asn
        435                 440                 445
Leu Thr Met Asp Gln Met Ala Thr Leu Ala Asn Leu Phe Gly Ile Glu
    450                 455                 460
Leu Ile Asp Ile Lys
465

<210> 12
<211> 1422
<212> DNA
<213> Artificial Sequence

<220>
<223> modified L-arabinose isomerase, codon-optimized

<400> 12
atgttgaaga ccccagatta cgaattctgg ttcgctgtcg gttcccaacc attgtacggt      60

ccagaagctt tggaacaagt cgaaaaggat ggtagaaagt tggtcgatgg tttgaacaag     120

aaggacttgc catacaaggt cgttttcaag ttggtcgcta ccactgctga ttccatcacc     180

aagttcatga aggatgctaa ctacaacgat aaggtcgctg gtgtcatgac ctggatgcat     240

accttctccc cagctaagaa ctggatcaga ggtacccaat tgttacaaaa gccattgtta     300

catttggcta cccaaatgtt ggatcatatc ccattcgata ccatcgactt cgattacatg     360

aacttgaacc aatccgctca tggtgataga gaatacgatt acatcaacgc tagattgggt     420

gtcccagaaa agatcgtcta cggttggtgg aacgatccag aagtccaaga agaaatcgct     480

gattggcaaa aggtcgctat cgcctacgat atgtccttca agatcaagat tgctagattc 540

ggtgatacca tgagagatgt cgctgttacc gaaggtgata aggtcgctgc ccaaatcaag 600

ttgggttgga ccgtcgatta ctggccagtc gctgaattgg tcgctgccgt caacgctgtc 660

tccgaagctg aaatcgatga acaatacaag agattggaag ataactacga catggtcgaa 720

ggtgataacg accatgataa gtacgtccat tccgtcagat accaattgag agaatacttg 780

ggtatcaagc atttcttgga tgaaaagggt tacgatgctt tcaccactaa cttccaagat 840

ttggaaggtt tggaacaatt gccaggtttg gctgtccaat tgttaatgat cgatggttac 900

ggtttcggtg ctgaaggtga tttcaagtcc gctggtttgt ctagattgtt aaagatcgct 960

gccgaaaaca aggaaaccgc tttcatggaa gattacacct tggatttgag agctggtcat 1020

caagctatca tgggttccca tatgttggaa gtcgatccaa ccttggcttc cgataagcca 1080

agagtcgaag ttcatccatt ggatatcggt gacaaggctg atccagctag attggtcttc 1140

accggttccg aaggtgaagg tatcgatgtc accttgtcct acttcgatga cggttacaag 1200

ttcatcgctt acgatgtcga ctgtgctaag ccagaaaagg aaatgccaaa cttgccagtc 1260

gctaagcaaa tgtggacccc aaagtgtggt ttgaagaaag gtgctaccgc ctggatgcat 1320

aacggtggtg gtcatcacac cgtcttgtcc atgaacttga ccatggatca aatggctacc 1380

ttggctaact tgttcggtat cgaattgatt gatatcaagt ga 1422

<210> 13
<211> 473
<212> PRT
<213> Artificial Sequence

<220>
<223> modified L-arabinose isomerase

<400> 13
Met Leu Lys Thr Pro Asp Tyr Glu Phe Trp Phe Ala Val Gly Ser Gln
1               5                   10                  15
Pro Leu Tyr Gly Pro Glu Ala Leu Glu Gln Val Glu Lys Asp Gly Arg
            20                  25                  30
Lys Leu Val Asp Gly Leu Asn Lys Lys Asp Leu Pro Tyr Lys Val Val
        35                  40                  45
Phe Lys Leu Val Ala Thr Thr Ala Asp Ser Ile Thr Lys Phe Met Lys
    50                  55                  60
Asp Ala Asn Tyr Asn Asp Lys Val Ala Gly Val Met Thr Trp Met His
65                  70                  75                  80
Thr Phe Ser Pro Ala Lys Asn Trp Ile Arg Gly Thr Gln Leu Leu Gln
                85                  90                  95
Lys Pro Leu Leu His Leu Ala Thr Gln Met Leu Asp His Ile Pro Phe
            100                 105                 110
Asp Thr Ile Asp Phe Asp Tyr Met Asn Leu Asn Gln Ser Ala His Gly
        115                 120                 125
Asp Arg Glu Tyr Asp Tyr Ile Asn Ala Arg Leu Gly Val Pro Glu Lys
    130                 135                 140
Ile Val Tyr Gly Trp Trp Asn Asp Pro Glu Val Gln Glu Glu Ile Ala

145                 150                 155                 160
Asp Trp Gln Lys Val Ala Ile Ala Tyr Asp Met Ser Phe Lys Ile Lys
                165                 170                 175
Ile Ala Arg Phe Gly Asp Thr Met Arg Asp Val Ala Val Thr Glu Gly
            180                 185                 190
Asp Lys Val Ala Ala Gln Ile Lys Leu Gly Trp Thr Val Asp Tyr Trp
        195                 200                 205
Pro Val Ala Glu Leu Val Ala Ala Val Asn Ala Val Ser Glu Ala Glu
    210                 215                 220
Ile Asp Glu Gln Tyr Lys Arg Leu Glu Asp Asn Tyr Asp Met Val Glu
225                 230                 235                 240
Gly Asp Asn Asp His Asp Lys Tyr Val His Ser Val Arg Tyr Gln Leu
                245                 250                 255
Arg Glu Tyr Leu Gly Ile Lys His Phe Leu Asp Glu Lys Gly Tyr Asp
            260                 265                 270
Ala Phe Thr Thr Asn Phe Gln Asp Leu Glu Gly Leu Glu Gln Leu Pro
        275                 280                 285
Gly Leu Ala Val Gln Leu Leu Met Ile Asp Gly Tyr Gly Phe Gly Ala
    290                 295                 300
Glu Gly Asp Phe Lys Ser Ala Gly Leu Ser Arg Leu Leu Lys Ile Ala
305                 310                 315                 320
Ala Glu Asn Lys Glu Thr Ala Phe Met Glu Asp Tyr Thr Leu Asp Leu
                325                 330                 335
Arg Ala Gly His Gln Ala Ile Met Gly Ser His Met Leu Glu Val Asp
            340                 345                 350
Pro Thr Leu Ala Ser Asp Lys Pro Arg Val Glu Val His Pro Leu Asp
        355                 360                 365
Ile Gly Asp Lys Ala Asp Pro Ala Arg Leu Val Phe Thr Gly Ser Glu
    370                 375                 380
Gly Glu Gly Ile Asp Val Thr Leu Ser Tyr Phe Asp Asp Gly Tyr Lys
385                 390                 395                 400
Phe Ile Ala Tyr Asp Val Asp Cys Ala Lys Pro Glu Lys Glu Met Pro
                405                 410                 415
Asn Leu Pro Val Ala Lys Gln Met Trp Thr Pro Lys Cys Gly Leu Lys
            420                 425                 430
Lys Gly Ala Thr Ala Trp Met His Asn Gly Gly Gly His His Thr Val
        435                 440                 445
Leu Ser Met Asn Leu Thr Met Asp Gln Met Ala Thr Leu Ala Asn Leu
    450                 455                 460
Phe Gly Ile Glu Leu Ile Asp Ile Lys
465                 470

<210> 14
<211> 1449
<212> DNA
<213> Artificial Sequence

<220>
<223> modified L-arabinose isomerase, N-terminal 6xhis-tag, codon
      optimized

<400> 14
atgcatcacc accatcatca cgagaactta tattttcaaa gcttgaagac cccagattac      60

gaattctggt tcgctgtcgg ttcccaacca ttgtacggtc cagaagcttt ggaacaagtc     120

gaaaaggatg gtagaaagtt ggtcgatggt ttgaacgctt ccggtaagtt gccatacaag     180

gtcgttttca agttggtcgc taccactgct gattccatca ccaagttcat gaaggatgct     240

aactacaacg ataaggtcgc tggtgtcatg acctggatgc ataccttctc cccagctaag     300

aactggatca gaggtaccca attgttacaa aagccattgt tacatttggc tacccaaatg 360

ttggatcata tcccattcga taccatcgac ttcgattaca tgaacttgaa ccaatccgct 420

catggtgata gagaatacga ttacatcaac gctagattgg gtgtcccaga aaagatcgtc 480

tacggttggt ggaacgatcc agaagtccaa gaagaaatcg ctgattggca aaaggtcgct 540

atcgcctacg atatgtcctt caagatcaag attgctagat tcggtgatac catgagagat 600

gtcgctgtta ccgaaggtga taaggtcgct gcccaaatca agttgggttg gaccgtcgat 660

tactggccag tcgctgaatt ggtcgctgcc gtcaacgctg tctccgaagc tgaaatcgat 720

gaacaataca agagattgga agataactac gacattgacc caaaggcttc tatggattcc 780

gtcagatacc aattgagaga atacttgggt atcaagcatt tcttggatga aaagggttac 840

gatgctttca ccactaactt ccaagatttg gaaggtttgg aacaattgcc aggtttggct 900

gtccaattgt taatgatcga tggttacggt ttcggtgctg aaggtgattt caagtccgct 960

ggtttgtcta gattgttaaa gatcgctgcc gaaaacaagg aaaccgcttt catggaagat 1020

tacaccttgg atttgagagc tggtcatcaa gctatcatgg gttcccatat gttggaagtc 1080

gatccaacct tggcttccga taagccaaga gtcgaagttc atccattgga tatcggtgac 1140

aaggctgatc cagctagatt ggtcttcacc ggttccgaag gtgaaggtat cgatgtcacc 1200

ttgtcctact tcgatgacgg ttacaagttc atcgcttacg atgtcgactg tgctaagcca 1260

gaaaaggaaa tgccaaactt gccagtcgct aagcaaatgt ggaccccaaa gtgtggtttg 1320

aagaaaggtg ctaccgcctg gatgcataac ggtggtggtc atcacaccgt cttgtccatg 1380

aacttgacca tggatcaaat ggctaccttg gctaacttgt tcggtatcga attgattgat 1440

atcaagtga 1449

<210> 15
<211> 482
<212> PRT
<213> Artificial Sequence

<220>
<223> modified L-arabinose isomerase, N-terminal 6xhis-tag

<400> 15
Met His His His His His His Glu Asn Leu Tyr Phe Gln Ser Leu Lys
1               5                   10                  15
Thr Pro Asp Tyr Glu Phe Trp Phe Ala Val Gly Ser Gln Pro Leu Tyr
            20                  25                  30
Gly Pro Glu Ala Leu Glu Gln Val Glu Lys Asp Gly Arg Lys Leu Val
        35                  40                  45
Asp Gly Leu Asn Ala Ser Gly Lys Leu Pro Tyr Lys Val Val Phe Lys
    50                  55                  60
Leu Val Ala Thr Thr Ala Asp Ser Ile Thr Lys Phe Met Lys Asp Ala
65                  70                  75                  80
Asn Tyr Asn Asp Lys Val Ala Gly Val Met Thr Trp Met His Thr Phe
                85                  90                  95

Ser Pro Ala Lys Asn Trp Ile Arg Gly Thr Gln Leu Leu Gln Lys Pro
            100                 105                 110
Leu Leu His Leu Ala Thr Gln Met Leu Asp His Ile Pro Phe Asp Thr
        115                 120                 125
Ile Asp Phe Asp Tyr Met Asn Leu Asn Gln Ser Ala His Gly Asp Arg
    130                 135                 140
Glu Tyr Asp Tyr Ile Asn Ala Arg Leu Gly Val Pro Glu Lys Ile Val
145                 150                 155                 160
Tyr Gly Trp Trp Asn Asp Pro Glu Val Gln Glu Glu Ile Ala Asp Trp
                165                 170                 175
Gln Lys Val Ala Ile Ala Tyr Asp Met Ser Phe Lys Ile Lys Ile Ala
            180                 185                 190
Arg Phe Gly Asp Thr Met Arg Asp Val Ala Val Thr Glu Gly Asp Lys
        195                 200                 205
Val Ala Ala Gln Ile Lys Leu Gly Trp Thr Val Asp Tyr Trp Pro Val
    210                 215                 220
Ala Glu Leu Val Ala Ala Val Asn Ala Val Ser Glu Ala Glu Ile Asp
225                 230                 235                 240
Glu Gln Tyr Lys Arg Leu Glu Asp Asn Tyr Asp Ile Asp Pro Lys Ala
                245                 250                 255
Ser Met Asp Ser Val Arg Tyr Gln Leu Arg Glu Tyr Leu Gly Ile Lys
            260                 265                 270
His Phe Leu Asp Glu Lys Gly Tyr Asp Ala Phe Thr Thr Asn Phe Gln
        275                 280                 285
Asp Leu Glu Gly Leu Glu Gln Leu Pro Gly Leu Ala Val Gln Leu Leu
    290                 295                 300
Met Ile Asp Gly Tyr Gly Phe Gly Ala Glu Gly Asp Phe Lys Ser Ala
305                 310                 315                 320
Gly Leu Ser Arg Leu Leu Lys Ile Ala Ala Glu Asn Lys Glu Thr Ala
                325                 330                 335
Phe Met Glu Asp Tyr Thr Leu Asp Leu Arg Ala Gly His Gln Ala Ile
            340                 345                 350
Met Gly Ser His Met Leu Glu Val Asp Pro Thr Leu Ala Ser Asp Lys
        355                 360                 365
Pro Arg Val Glu Val His Pro Leu Asp Ile Gly Asp Lys Ala Asp Pro
    370                 375                 380
Ala Arg Leu Val Phe Thr Gly Ser Glu Gly Glu Gly Ile Asp Val Thr
385                 390                 395                 400
Leu Ser Tyr Phe Asp Asp Gly Tyr Lys Phe Ile Ala Tyr Asp Val Asp
                405                 410                 415
Cys Ala Lys Pro Glu Lys Glu Met Pro Asn Leu Pro Val Ala Lys Gln
            420                 425                 430
Met Trp Thr Pro Lys Cys Gly Leu Lys Lys Gly Ala Thr Ala Trp Met
        435                 440                 445
His Asn Gly Gly Gly His His Thr Val Leu Ser Met Asn Leu Thr Met
    450                 455                 460
Asp Gln Met Ala Thr Leu Ala Asn Leu Phe Gly Ile Glu Leu Ile Asp
465                 470                 475                 480
Ile Lys

<210> 16
<211> 1461
<212> DNA
<213> Artificial Sequence

<220>
<223> modified L-arabinose isomerase, N-terminal 6xhis-tag, codon
      optimized

<400> 16
atgcatcacc accatcatca cgagaactta tattttcaaa gcttgaagac cccagattac       60

gaattctggt tcgctgtcgg ttcccaacca ttgtacggtc cagaagcttt ggaacaagtc 120

gaaaaggatg gtagaaagtt ggtcgatggt ttgaacaaga aggacttgcc atacaaggtc 180

gttttcaagt tggtcgctac cactgctgat tccatcacca agttcatgaa ggatgctaac 240

tacaacgata aggtcgctgg tgtcatgacc tggatgcata ccttctcccc agctaagaac 300

tggatcagag gtacccaatt gttacaaaag ccattgttac atttggctac ccaaatgttg 360

gatcatatcc cattcgatac catcgacttc gattacatga acttgaacca atccgctcat 420

ggtgatagag aatacgatta catcaacgct agattgggtg tcccagaaaa gatcgtctac 480

ggttggtgga acgatccaga agtccaagaa gaaatcgctg attggcaaaa ggtcgctatc 540

gcctacgata tgtccttcaa gatcaagatt gctagattcg gtgataccat gagagatgtc 600

gctgttaccg aaggtgataa ggtcgctgcc caaatcaagt tgggttggac cgtcgattac 660

tggccagtcg ctgaattggt cgctgccgtc aacgctgtct ccgaagctga aatcgatgaa 720

caatacaaga gattggaaga taactacgac atggtcgaag gtgataacga ccatgataag 780

tacgtccatt ccgtcagata ccaattgaga gaatacttgg gtatcaagca tttcttggat 840

gaaaagggtt acgatgcttt caccactaac ttccaagatt tggaaggttt ggaacaattg 900

ccaggtttgg ctgtccaatt gttaatgatc gatggttacg gtttcggtgc tgaaggtgat 960

ttcaagtccg ctggtttgtc tagattgtta aagatcgctg ccgaaaacaa ggaaaccgct 1020

ttcatggaag attacacctt ggatttgaga gctggtcatc aagctatcat gggttcccat 1080

atgttggaag tcgatccaac cttggcttcc gataagccaa gagtcgaagt tcatccattg 1140

gatatcggtg acaaggctga tccagctaga ttggtcttca ccggttccga aggtgaaggt 1200

atcgatgtca ccttgtccta cttcgatgac ggttacaagt tcatcgctta cgatgtcgac 1260

tgtgctaagc cagaaaagga aatgccaaac ttgccagtcg ctaagcaaat gtggacccca 1320

aagtgtggtt tgaagaaagg tgctaccgcc tggatgcata acggtggtgg tcatcacacc 1380

gtcttgtcca tgaacttgac catggatcaa atggctacct tggctaactt gttcggtatc 1440

gaattgattg atatcaagtg a 1461

<210> 17
<211> 486
<212> PRT
<213> Artificial Sequence

<220>
<223> modified L-arabinose isomerase, N-terminal 6xhis-tag

<400> 17
Met His His His His His His Glu Asn Leu Tyr Phe Gln Ser Leu Lys
1               5                   10                  15
Thr Pro Asp Tyr Glu Phe Trp Phe Ala Val Gly Ser Gln Pro Leu Tyr

20                  25                  30
Gly Pro Glu Ala Leu Glu Gln Val Glu Lys Asp Gly Arg Lys Leu Val
        35                  40                  45
Asp Gly Leu Asn Lys Lys Asp Leu Pro Tyr Lys Val Val Phe Lys Leu
    50                  55                  60
Val Ala Thr Thr Ala Asp Ser Ile Thr Lys Phe Met Lys Asp Ala Asn
65                  70                  75                  80
Tyr Asn Asp Lys Val Ala Gly Val Met Thr Trp Met His Thr Phe Ser
                85                  90                  95
Pro Ala Lys Asn Trp Ile Arg Gly Thr Gln Leu Leu Gln Lys Pro Leu
            100                 105                 110
Leu His Leu Ala Thr Gln Met Leu Asp His Ile Pro Phe Asp Thr Ile
        115                 120                 125
Asp Phe Asp Tyr Met Asn Leu Asn Gln Ser Ala His Gly Asp Arg Glu
    130                 135                 140
Tyr Asp Tyr Ile Asn Ala Arg Leu Gly Val Pro Glu Lys Ile Val Tyr
145                 150                 155                 160
Gly Trp Trp Asn Asp Pro Glu Val Gln Glu Glu Ile Ala Asp Trp Gln
                165                 170                 175
Lys Val Ala Ile Ala Tyr Asp Met Ser Phe Lys Ile Lys Ile Ala Arg
            180                 185                 190
Phe Gly Asp Thr Met Arg Asp Val Ala Val Thr Glu Gly Asp Lys Val
        195                 200                 205
Ala Ala Gln Ile Lys Leu Gly Trp Thr Val Asp Tyr Trp Pro Val Ala
    210                 215                 220
Glu Leu Val Ala Ala Val Asn Ala Val Ser Glu Ala Glu Ile Asp Glu
225                 230                 235                 240
Gln Tyr Lys Arg Leu Glu Asp Asn Tyr Asp Met Val Glu Gly Asp Asn
                245                 250                 255
Asp His Asp Lys Tyr Val His Ser Val Arg Tyr Gln Leu Arg Glu Tyr
            260                 265                 270
Leu Gly Ile Lys His Phe Leu Asp Glu Lys Gly Tyr Asp Ala Phe Thr
        275                 280                 285
Thr Asn Phe Gln Asp Leu Glu Gly Leu Glu Gln Leu Pro Gly Leu Ala
    290                 295                 300
Val Gln Leu Leu Met Ile Asp Gly Tyr Gly Phe Gly Ala Glu Gly Asp
305                 310                 315                 320
Phe Lys Ser Ala Gly Leu Ser Arg Leu Leu Lys Ile Ala Ala Glu Asn
                325                 330                 335
Lys Glu Thr Ala Phe Met Glu Asp Tyr Thr Leu Asp Leu Arg Ala Gly
            340                 345                 350
His Gln Ala Ile Met Gly Ser His Met Leu Glu Val Asp Pro Thr Leu
        355                 360                 365
Ala Ser Asp Lys Pro Arg Val Glu Val His Pro Leu Asp Ile Gly Asp
    370                 375                 380
Lys Ala Asp Pro Ala Arg Leu Val Phe Thr Gly Ser Glu Gly Glu Gly
385                 390                 395                 400
Ile Asp Val Thr Leu Ser Tyr Phe Asp Asp Gly Tyr Lys Phe Ile Ala
                405                 410                 415
Tyr Asp Val Asp Cys Ala Lys Pro Glu Lys Glu Met Pro Asn Leu Pro
            420                 425                 430
Val Ala Lys Gln Met Trp Thr Pro Lys Cys Gly Leu Lys Lys Gly Ala
        435                 440                 445
Thr Ala Trp Met His Asn Gly Gly Gly His His Thr Val Leu Ser Met
    450                 455                 460
Asn Leu Thr Met Asp Gln Met Ala Thr Leu Ala Asn Leu Phe Gly Ile
465                 470                 475                 480
Glu Leu Ile Asp Ile Lys
                485

## Description

The present invention relates to a temperature optimized L-arabinose isomerase with a high catalytic activity within feedstocks comprising a high concentration of divalent metal ions, a nucleic acid sequence encoding the inventive L-arabinose isomerase, a vector comprising the nucleic acid sequence encoding the inventive L-arabinose isomerase, a composition containing the inventive L-arabinose isomerase, a yeast cell comprising the inventive L-arabinose isomerase, the use of the inventive L-arabinose isomerase, the composition or the yeast cell for the fermentation of feedstock with a high content of divalent metal ions.

Large-scale consumption of traditional, fossil fuels (petroleum-based fuels) in recent decades has contributed to high levels of pollution. This, along with the realisation that the world stock of fossil fuels is limited has stimulated new initiatives to investigate the feasibility of alternative fuels such as biomass-derived ethanol.

Although biomass-derived ethanol may be produced by the fermentation of hexose sugars obtained from agricultural products such as cane sugar and corn starch, such feedstocks are costly and industrial scale production will also severely affect global food supply.

Newer processes therefore focus on the conversion of alternative biomass material known as "lignocellulosic feedstock" such as straw and sugar cane bagasse, the so called second-generation (2G) production of biofuels. Even though lignocellulosic feedstock is available in high quantities and low in price, the feedstock is more complex and an efficient and economical conversion to ethanol is difficult.

Within the state of the art, the conversion of lignocellulosic feedstock to ethanol is known to afford several process steps, starting from a mechanical comminution by e.g. milling, a pretreatment of the comminuted material by e.g. applying steam and pressure, an enzymatic hydrolyzation of the pretreated material by using an enzymatic composition comprising cellulose and hemicellulose degrading enzymes (e.g. produced a filamentous fungus such as *Trichoderma reesei*) and a subsequent fermentation of the hydrolysate to ethanol by a yeast.

In addition to a large amount of hexose sugars, hydrolysate of lignocellulosic feedstock also contains a considerable amount of pentose sugars, including L-arabinose. For an economically feasible fuel production both hexose and pentose sugars must be fermented to ethanol.

The yeast *Saccharomyces cerevisiae* most commonly used in such processes is robust and well adapted for ethanol production, but it is unable to convert arabinose, in particular L-arabinose. Also, no naturally-occurring organisms are known which can ferment both, hexose and pentose sugars, to ethanol with a high ethanol yield and a high ethanol productivity.

Another obstacle is the high content of impurities and coproducts within the hydrolysate which interfere the fermentation process. A high content of salts leading to a high content of divalent metal ions is of particular concern and there are a number of new methods proposed for removing or limiting these impurities and coproducts. For example purification of the hydrolysate with membranes and ion exchange technology was recently disclosed. However, these purification steps significantly add to the cost of using lignocellulosic feedstock.

In addition, yeasts and enzymes used in such fermentation processes need to show a consistently optimal performance at a temperature of at least 30 °C to enable flexible process adaptions in particular during fermentation but also to limit additional cooling of the hydrolysate before entering the fermentation process to a minimum.

There is therefore a need for newly developed enzymes capable of converting L-arabinose to sugars which can be fermented by a yeast such as *Saccharomyces cerevisiae* to ethanol to enable a commercially-viable ethanol production from lignocellulosic feedstocks by overcoming the above referenced obstacles known from the state of the art.

The inventors of the present invention have therefore set themselves the task to develop novel L-arabinose isomerases which allow the efficient conversion of L-arabinose to L-ribulose, which is an essential intermediate for producing further products such as ethanol. In addition, a high tolerance of divalent metal ions content within the medium was requested. Further, the novel L-arabinose isomerase should also show an optimized performance at a wider temperature range. Finally, the L-arabinose isomerase should be efficient either applied as a separate enzyme or when cloned in the yeast cell.

The inventors of the present invention have now surprisingly found that this task can be solved by temperature optimized L-arabinose isomerase mutants with a high catalytic activity within feedstocks comprising a high concentration of divalent metal ions, having a sequence identity of at least 97.0 % to SEQ. ID NO:2.

In the following, the elements of the present invention will be described in more detail. These elements are listed with specific embodiments, however, it should be understood that they may be combined in any manner and in any number to create additional embodiments. The variously described examples and embodiments should not be construed to limit the present invention to only the explicitly described embodiments. This description should be understood to support and encompass embodiments which combine the explicitly described embodiments with any number of the disclosed elements. Furthermore, any permutations and combinations of all described elements in this application should be considered disclosed by the description of the present application unless the context indicates otherwise.

Throughout this specification and the claims, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example"), provided herein is intended merely to better illustrate the invention and does not pose a limitation on the scope of the invention otherwise claimed. No language in the specification should be construed as indicating any non-claimed element essential to the practice of the invention.

The nomenclature of amino acids, peptides, nucleotides and nucleic acids within the present application follows the suggestions of IUPAC. Generally, amino acids are named within this document according to the one letter code.

The terms "DNA" and "RNA" are well known to a person skilled in the art. While DNA contains deoxyribose, RNA contains ribose (in deoxyribose there is no hydroxyl group attached to the pentose ring in the 2' position). The complementary base to adenine is not thymine, as it is in DNA, but rather uracil, which is an unmethylated form of thymine.

If not otherwise specified, within the present invention the term "mutated" is to be understood as "substituted", "deleted" or "inserted". If not otherwise specified, the term "mutation" is to be understood as "substitution", "deletion" or "insertion". Substitutions are classified as transitions where a purine is exchanged by a purine (A <-> G) or a pyrimidine by a pyrimidine (C <->T) or transversions where a purine is exchanged by a pyridine and vice versa (C/T <-> A/G). Insertions add one or more additional nucleotides (A, C, T or G) into an oligonucleotide. The removal of one or more nucleotides from the DNA is called deletion.

The sequence identity between two amino acid sequences is determined by global alignment using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are the standard settings (EBLOSUM62 scoring matrix, gap open penalty of 10, gap extension penalty of 0.5). The output of Needle labeled "identity" is used as the percent identity.

Within the present invention, the term "L-arabinose isomerase" is to be understood as an enzyme belonging to the class EC 5.3.1.4. The L-arabinose isomerase according to the present invention is an enzyme that catalyzes the chemical reaction from L-arabinose to L-ribulose. This enzyme belongs to the family of isomerases, specifically those intramolecular oxidoreductases interconverting aldoses and ketoses. The term "L-arabinose isomerase" and enzymes belonging to class EC 5.3.1.4 are well known to a person skilled in the art, who is able to identify and differentiate an L-arabinose isomerase from other enzymes.

Within the present invention, the term "temperature optimized L-arabinose isomerase" refers to L-arabinose isomerases which show a catalytic activity of at least 28.5 kat/mol enzyme within a temperature range of from 30 °C to 50 °C during an incubation of 20 minutes at a pH of 7 and on a substrate containing 400 mM L-arabinose and comprising 1 mM Ca²⁺ or 1 mM Mg²⁺. The term "kat" (abbreviation for katal) is the unit of catalytic activity in the International System of Units (SI). It quantifies the catalytic activity of enzymes (that is, measuring the enzymatic activity level in enzyme catalysis).

Within the present invention, the term "high catalytic activity" refers to a catalytic activity of at least 28.5 kat/mol enzyme within a temperature range of from 30 °C to 50 °C during an incubation of 20 minutes at a pH of 7 and on a substrate containing 400 mM L-arabinose and comprising 1 mM Ca²⁺ or 1 mM Mg²⁺.

Within the present invention, the term feedstock is to be understood as referring to any feedstock comprising at least 0.5 g/l L-arabinose, at least 5 g/l of D-glucose and shows a content of at least 2 mM of divalent metal ions. Exemplary suitable feedstocks comprise from 0.5 to 15 g/l L-arabinose, from 5 to 200 g/l of D-glucose and show a content of from 2 to 50 mM of divalent metal ions. Particularly suitable feedstocks comprise from 0.5 to 10 g/l L-arabinose, from 5 to 150 g/l of D-glucose, from 5 to 60 g/l D-xylose and show a total content of from 5 to 30 mM of Mg²⁺ and Ca²⁺ ions. Further particularly preferred feedstocks are hydrolysates from cellulosic and/or lignocellulosic biomass such as hydrolysate from cereal straw and/or husks, sugar cane bagasse, switch grass, rice bran, corn stover, paper, raw paper pulp, waste-paper, wood, agricultural residues, forestry residues, municipal solid wastes hull products and mixtures thereof. The production of such a hydrolysate is known to a person skilled in the art and for exampled described within EP 2015723515 or WO 2017042019 which are herein incorporated by reference.

Within the present invention, the term "high concentration of divalent metal ions" is to be understood as a concentration of divalent metal ions of at least 2 mM, at least 5 mM, at least 10 mM or at least 15 mM. A concentration of divalent metal ions of from 2 to 50 mM, from 2 to 45 mM or from 5 to 35 mM is also within the scope of the present invention. Particularly suitable feedstocks comprise Mn²⁺, Mg²⁺ and/or Ca²⁺ in a concentration of from 1 to 40 mM, of from 2 to 35 mM or from 5 to 30 mM. The term "divalent metal ions" is well known to a person skilled in the art and to be understood as comprising Ca²⁺, Mn²⁺, Mg²⁺, Ba²⁺, CU²⁺, Zn²⁺.

The temperature optimized L-arabinose isomerase according to the present invention has a sequence identity of at least 97.0 % to SEQ ID NO: 2, wherein L-arabinose isomerases with a sequence identity of at least 97.5, at least 98.0 %, at least 98.5 %, at least 99.0 %, at least 99.5 or at least 99.9 % are particularly suitable for the inventive purpose.

Further particularly advantageous temperature optimized L-arabinose isomerases according to the present invention show a sequence identity of at least 97.0 % to SEQ. ID NO: 2 and further comprise SEQ ID NO: 4 with at least one mutation selected from the group consisting of deletions and substitutions at position 40, 41, 42, 43, 239, 240, 241, 242, 243, 244, 246, 247, 248, 249, 250 and 251. Within particularly advantageous temperature optimized isomerase L-arabinoses this at least one mutation is a substitution selected from the group consisting of substitutions at position 40, 41, 43, 239, 246, 247, 248, 249, 250 and 251, wherein it is even more particularly advantageous to select the at least one substitution from the group consisting of A40K, S41K, K43D, M239I, H246P, D247K, K248A, Y249S, V250M and H251D. Within other particularly advantageous temperature optimized L-arabinose isomerases this at least one mutation is a deletion selected from the group consisting of deletions at position 42, 240, 241, 242, 243 and 244, wherein it is even more advantageous to select the at least one deletion from the group consisting of G42, V240, E241, G242, D243 and N244. Combinations of these specified mutations are also within the scope of the present invention.

Other particularly advantageous temperature optimized L-arabinose isomerases according to the present invention show a sequence identity of at least 97.0 % to SEQ ID NO: 2 and further comprise SEQ ID NO: 4 with at least one substitution at position 40, 41 and 43 and/or at position 239, 246, 247, 248, 249, 250 and 251. Further particularly advantageous temperature optimized L-arabinose isomerases according to the present invention show a sequence identity of at least 97.0 % to SEQ ID NO: 2 and further comprise SEQ ID NO: 4 with at least one deletion at position 42 and/or at position 240, 241, 242, 243 and 244. Combinations of these specified mutations are also within the scope of the present invention.

Other particularly advantageous temperature optimized L-arabinose isomerases according to the present invention are selected from the group consisting of SEQ ID NO: 6, SEQ ID NO: 8, SEQ ID NO: 11, SEQ ID NO: 13, SEQ ID NO: 15 and SEQ ID NO: 17.

Other particularly advantageous temperature optimized L-arabinose isomerases according to the present invention have a sequence identity of at least 97.0 % to SEQ. ID NO: 2 and a catalytic activity of at least 28.5 kat/mol enzyme within feedstocks of an L-arabinose content of 400 mM and a content of MgCl₂ of 5 mM when incubated at 30 °C for 20 minutes at pH 7, wherein L-arabinose isomerases with a catalytic activity of at least 30.0 kat/mol enzyme and a content of 5 mM CaCl₂ are also advantageous.

Within another embodiment, the present invention further provides a nucleic acid encoding the temperature optimized L-arabinose isomerase according to the present invention.

Within another embodiment, the present invention further provides vectors comprising the nucleic acid of the present invention. Examples for episomally maintained vectors are derivatives of bacterial plasmids, yeast plasmids, centromer based linear DNA, constructs of viral origin like SV40, phage DNA, fungal ARS based DNA-vehicles, baculovirus, vaccinia, adenovirus, fowl pox virus, and pseudorabies as well as vectors derived from combinations of plasmids and phage or viral DNA. A suitable expression vector according to the present invention may comprise one or more genetic elements representing promotor sequences, transcription initiation sites, elements for the initiation of translation, and functional elements for protein export that are translationally coupled to the nucleic acid according to the present invention. In addition to the inventive nucleic acid, the vector according to the present invention may encode more than one polypeptide including more than one L-arabinose isomerase or may encode a fusion polypeptide comprising the L-arabinose isomerase according to the invention. The vector according to the present invention may be episomally maintained in the host cell or integrated into the chromosome of the host.

Within a further embodiment, the present invention provides a composition comprising the temperature optimized L-arabinose isomerase according to the present invention. The composition may be a liquid or dry composition. Liquid compositions according to the present invention may only contain the inventive L-arabinose isomerase, preferably in a highly purified form. Usually, however, a stabilizer such as glycerol, sorbitol or mono propylene glycol is also contained. The liquid composition may also comprise other additives, such as salts, sugars, preservatives, pH-adjusting agents and/or proteins. Typical liquid compositions are aqueous or oil-based slurries. Dry compositions may be spray dried compositions, in which case the composition need not contain anything more than the inventive L-arabinose isomerase in a dry form. Usually, however, dry compositions are so-called granulates which may readily be mixed with the substrate/feedstock. Agglomeration granulates coated with the inventive arabinose isomerase can be prepared using agglomeration techniques in a high shear mixer. Absorption granulates can be prepared by having cores of a carrier material to absorb/be coated by inventive L-arabinose isomerase. The carrier material may be a biocompatible non-biodegradable. Typical filler materials used in agglomeration techniques include salts, such as disodium sulphate. Other fillers are kaolin, talc, magnesium, aluminum, silicate and cellulose fibers. Optionally, binders such as dextrins are also included in agglomeration granulates.

The composition may further comprise at least one enzyme selected from the group consisting cellulases such as endoglucanases, cellobiohydrolases and beta-glucosidases, hemicellulases such as xylanases, xylosidases, mannanases and mannosidases, pectinases, ribulokinases and ribulose-5-phosphate-4-epimerases.

Within a further embodiment, the present invention provides a yeast cell comprising the temperature optimized L-arabinose isomerase according to the present invention. Within a particularly advantageous embodiment the yeast cell according to the present invention is selected from the group consisting of *Saccharomyces, Kluyveromyces, Schizosaccharomyces, Candida, Yarrowia, Komagataella, Pichia* and *Hansenula* wherein a yeast cell selected from the group consisting of *Saccharomyces cerevisiae, Saccharomyces bayanus, Saccharomyces uravum, Saccharomyces pastorianus, Saccharomyces kudriavzevii, Saccharomyces mikatae, Saccharomyces carlsbergensis, Schizosaccharomyces pombe, Kluyveromyces marxianus, Yarrowina lipolytica, Hansenula polymorpha, Pichia angusta, Komagataella pastoris* and *Pichia pastoris* is even more suitable for the inventive purpose.

The yeast cell according to the present invention may comprise one or more vectors according to the present invention. In addition or alternatively, the nucleic acid is integrated in the genome of the yeast cell.

Within a further embodiment, the present invention provides the use of the temperature optimized L-arabinose isomerase or of the inventive composition comprising the temperature optimized L-arabinose isomerase or the inventive yeast cell comprising the temperature optimized L-arabinose isomerase within a fermentation process of feedstock with a high content of divalent metal ions. Such fermentation processes are well known to a person skilled in the art.

Feedstocks comprising arabinose, particularly L-arabinose, can also serve as precursors to platform chemicals that can be converted to a wide variety of products. Continued advances in sugar production and conversion are key factors in the future of biomass-based fuels and chemicals. It is therefore particularly suitable that the temperature optimized L-arabinose isomerase, the yeast cell and/or the composition according to the present invention is used for a fermentation process for the production of a fermentation product such as e.g. ethanol, butanol, lactic acid, 3 -hydroxy-propionic acid, acrylic acid, acetic acid, succinic acid, citric acid, malic acid, fumaric acid, itaconic acid, an amino acid, 1,3-propane-diol, ethylene, glycerol, a β-lactam antibiotic, such as Penicillin and fermentative derivatives thereof.

### Generally preferred embodiments

In the following, generally preferred embodiments of the present invention are listed. These embodiments illustrate particularly advantageous embodiments of the present inventions and do not limit the scope of the present invention and claims in any respect.

### Generally preferred embodiment A

Temperature optimized L-arabinose isomerase with a high catalytic activity within feedstocks comprising a high concentration of divalent metal ions, having a sequence identity of at least 97.0 % to SEQ. ID NO:2 and comprising SEQ. ID NO: 4 with a substitution at position 40, 41 and 43 and/or at position 239, 246, 247, 248, 249, 250 and 251.

### Generally preferred embodiment B

Temperature optimized L-arabinose isomerase according to generally preferred embodiment A with a deletion at position 42 and/or at position 240, 241, 242, 243 and 244.

### Generally preferred embodiment C

Temperature optimized L-arabinose isomerase according to generally preferred embodiment A or B, wherein the L-arabinose isomerases show a catalytic activity of at least 28.5 kat/mol enzyme within feedstocks of an L-arabinose content of 400 mM and a content of MgCl₂ of 5 mM when incubated at 30 °C for 20 minutes at pH 7.

### Generally preferred embodiment D

Temperature optimized L-arabinose isomerase according to generally preferred embodiment C, wherein the L-arabinose isomerases show a catalytic activity of at least 30.0 kat/mol enzyme within feedstocks of an L-arabinose content of 400 mM and a content of CaCl₂ of 5 mM when incubated at 30 °C for 20 minutes at pH 7.

### Generally preferred embodiment E

Temperature optimized L-arabinose isomerase according to generally preferred embodiment A or B, wherein the L-arabinose isomerases show a catalytic activity of at least 28.5 kat/mol enzyme within feedstocks of an L-arabinose content of 400 mM and a content of MgCl₂ of 5 mM when incubated at 30 °C for 20 minutes at pH 7.

### Generally preferred embodiment F

Temperature optimized L-arabinose isomerase according to generally preferred embodiment A or B, wherein the L-arabinose isomerases show a catalytic activity of at least 28.5 kat/mol enzyme within feedstocks containing from 0.5 to 15 g/l L-arabinose, from 5 to 200 g/l of D-glucose and a content of from 2 to 50 mM of divalent metal ions when incubated at 30 °C for 20 minutes at pH 7.

### Generally preferred embodiment G

Temperature optimized L-arabinose isomerase according to generally preferred embodiment A or B, wherein the L-arabinose isomerases show a catalytic activity of at least 28.5 kat/mol enzyme within feedstocks containing from 0.5 to 10 g/l L-arabinose, from 5 to 100 g/l of D-glucose, from 5 to 50 g/l D-xylose and a total content of from 5 to 30 mM of Mg²⁺ and Ca²⁺ ions when incubated at 30 °C for 20 minutes at pH 7 and wherein the feedstock is a hydrolysate of lignocellulose containing biomass such as cereal straw, bagasse, cornstover, rice bran, rice hulls, rice straw or mixtures thereof.

### Generally preferred embodiment H

Temperature optimized L-arabinose isomerase according to any of generally preferred embodiment A to G, having a sequence identity of at least 98.5 % to SEQ ID NO:2 and comprising SEQ. ID NO: 4 with a substitution at position 40, 41 and 43 and/or at position 239, 246, 247, 248, 249, 250 and 251.

### Generally preferred embodiment I

Temperature optimized L-arabinose isomerase according to any of generally preferred embodiment A to G, having a sequence identity of at least 99.5 % to SEQ. ID NO:2 and comprising SEQ ID NO: 4 with at least one deletion at position 42 and/or at position 240, 241, 242, 243 and 244.

### Generally preferred embodiment J

Nucleic acid sequence encoding a temperature optimized L-arabinose isomerase according to any of generally preferred embodiments A to G.

### Generally preferred embodiment K

Composition containing a temperature optimized L-arabinose isomerase according to any of generally preferred embodiments A to G.

### Generally preferred embodiment L

Yeast cell comprising a temperature optimized L-arabinose isomerase according to any of generally preferred embodiments A to G.

### Generally preferred embodiment M

Use of a temperature optimized L-arabinose isomerase according to any of generally preferred embodiments A to G, a composition according to generally preferred embodiment I or a yeast cell according to generally preferred embodiment J within a fermentation process of feedstocks containing from 0.5 to 10 g/l L-arabinose, from 5 to 100 g/l of D-glucose, from 5 to 50 g/l D-xylose and a total content of from 5 to 30 mM of Mg²⁺ and Ca²⁺ ions and wherein the feedstock is a hydrolysate of lignocellulose containing biomass such as cereal straw, bagasse, cornstover, rice bran, rice hulls, rice straw or mixtures thereof.

### Generally preferred embodiment N

Use according to generally preferred embodiment M, wherein the fermentation process is a fermentation process for the production of a fermentation product such as e.g. ethanol, butanol, lactic acid, 3 -hydroxy-propionic acid, acrylic acid, acetic acid, succinic acid, citric acid, malic acid, fumaric acid, itaconic acid, an amino acid, 1,3-propane-diol, ethylene, glycerol, a β-lactam antibiotic, such as Penicillin and fermentative derivatives thereof.

### Examples and Figures

In the following the present invention is described by the examples and figures. The examples and figures are considered for illustrative purpose only and do not limit the scope of the present invention and claims in any respect.

### Brief description of the figures

- Fig. 1: shows the comparison of the enzymatic activity at 30 °C for substrates containing 1 or 5 mM MnCl₂, MgCl₂ or CaCl₂ and 400 mM L-arabinose for SEQ ID NO: 8 (state of the art L-arabinose isomerase) and SEQ ID NO: 6 (L-arabinose isomerase according to the invention)
- Fig. 2: shows the comparison of the enzymatic activity at 30 °C and 50 °C for substrates containing 1 or 5 mM MgCl₂ or CaCl₂ and 400 mM L-arabinose for SEQ ID NO: 8 (state of the art L-arabinose isomerase) and SEQ ID NO: 6 (L-arabinose isomerase according to the invention).

### Example 1

Cloning of vectors for expression of L-arabinose isomerase genes in *Escherichia coli* Methods for manipulation of nucleic acid molecules are generally known to the skilled person in the field and are here introduced by reference (1. Molecular cloning: a laboratory manual, Michael R. Green and Joseph Sambrook, 4th edition; 2. Current Protocols in Molecular Biology, ISSN: 1934-3639).

SEQ ID NO: 1 and 3, coding for SEQ ID NO: 2 and 4, respectively, were synthesized by Geneart (Regensburg, Germany) and then used as template in PCR reactions for cloning. A sequence coding for a N-terminal 6xHis-tag followed by a TEV protease site (ENLYFQS) was added to the ORF in two successive PCRs. The resulting open reading frames are SEQ ID NO: 5 and SEQ ID NO: 7, respectively. The corresponding amino acid sequences are SEQ ID NO: 6 and SEQ ID NO: 8. For the first PCR reaction, primer pairs were designed to match the 5' and 3' ends of SEQ ID NO: 1 and 3, respectively. The forward primer (binding to the 5' end of the respective ORF) has a 5' extension containing the sequence of the TEV protease site and a part of the 6xHis-tag sequence (12 bp). The reverse primer has a 5' extension suitable for Gibson cloning into the target vector. The forward primer for the second reaction was designed to bind the 5' extension of the first forward primer and has a 5'extension containing the residual 6xHis-tag sequence and a sequence allowing Gibson cloning into the target vector. The reverse primer for the first and second reaction are identical. Both PCR reactions were set up using Q5 High Fidelity DNA Polymerase (HF-Buffer system) following the recommendations of the supplier for dNTP, primer and buffer concentrations. The amplification of the PCR products was done in an Eppendorf Thermocycler using the standard program for Phusion Polymerase (98°C, 30 sec initial denaturation followed by 30 cycles of 98°C, 20 sec - 60°C, 20 sec - 72°C, 30 sec and a final elongation phase at 72°C for 5 minutes. The PCR products of expected size were purified by preparative, ethidium bromide stained TAE-Agarose gel electrophoresis and recovered from the Gel using the Promega Wizard SV-PCR and Gel Purification Kit.

Plasmid pET-22b was linearized by digestion with restriction endonucleases *Hin*dIII and *Nde*I and the digested fragment separated by agarose gel electrophoresis. The linearized vector-backbone was recovered from the gel following the instructions of the Promega Wizard SV-PCR and Gel Purification Kit. The amplified PCR product was cloned into the digested vector-backbone using a Gibson cloning methods (NEBuilder) according to manufacturer's indications (vector-insert ration 1:3, 0.1 pmol total DNA, 20 µl reaction). Transformation was performed into competent *E. coli* Mach1 cells according to the supplier's protocol. Transformants were grown over night on LB-Ampicillin plates and tested for correct plasmid by plasmid mini-prep and control digestion as well as DNA sequencing. A larger quantity of plasmid DNA was prepared from a confirmed clone using the Promega PureYield™ Plasmid Midiprep System.

### Expression of L-arabinose isomerase in E. coli and L-arabinose isomerase purification

The L-arabinose isomerases were expressed in *E. coli* Rosetta™ cells under IPTG (Isopropyl β-D-1-thiogalactopyranoside) inducible promoter by using standard molecular biology technics. A preculture of 200 ml Lysogeny Broth containing 34 µg/mL Chloramphenicol and 100 µg/ml Ampicillin was inoculated directly with the transformed *E. coli* cells and grown over-night at 37 °C and 250 rpm shaking. Eight main cultures of 250 ml Terrific Broth with 100 µg/ml ampicillin in 2L shake flasks were inoculated to an OD₆₀₀ of 0.1 with the over-night culture and incubated at 37 °C and 250 rpm shaking. When the cultures reached an OD₆₀₀ of 1.0, the temperature was reduced to 20 °C and the expression of the Al induced by addition of 1 mM IPTG. After 21.5 hours cells were harvested by centrifugation (10 min, 10,000 x g). The pellets were washed once with saline (0.9 % NaCl) and combined into one tube. The pellet was then stored at -20 °C until cell lysis.

For cell lysis, the cell pellet was thawed, resuspended in 70 ml binding buffer (20 mM Trizma base, 500 mM NaCl, 20 mM Imidazole, pH 7.4) and protease inhibitor (cOmplete™, EDTA-free Protease Inhibitor Cocktail), Lysozyme and Benzonase Nuclease (Sigma-Aldrich, 62970 and E1014) were added. The cell suspension was incubated on ice for 30 minutes. The cells were then lysed by ultrasonification while being kept on ice with the following settings: 4 x 1 min, amplitude 70 %, cycle 0.6, with 1 min breaks in between. The lysed cells were centrifuged for 60 min at 20,000 x g at 4°C. The cleared supernatant was filtered (0.2 µm) and used for purification of the respective Al.

Purification was done using an Äkta explorer 900 and a 5 mL HisTrap HP column (GE life sciences). The column was equilibrated with binding buffer and the sample then loaded onto the column. The column was washed with binding buffer until the absorption at 280 nm had normalized. Elution was done with a gradient over 10 column volumes (50 mL, flow 3 ml/min, 17 minutes) with elution buffer (20 mM Trizma base, 500 mM NaCl, 500 mM Imidazole, pH 7.4). Fractions of 2 ml were collected and later analyzed by SDS-PAGE (Biorad Criterion XT 4-12 % Bis-Tris) according manufacturer's instructions. Fractions containing the respective Al protein were pooled and, to remove divalent cations from the protein, were dialyzed at 4°C against 2x 2L of dialysis buffer (50 mM HEPES, pH 7.0, 10 mM EDTA) over 96h with one buffer change. Al concentration was determined photometrically via absorption at 280 nm.

Proteins with SEQ ID NO: 6 and SEQ ID NO: 8 were obtained at concentrations of 15.4 and 12.3 g/L, respectively.

### Measurement of L-arabinose isomerase activity

Measurements to determine the catalytic activity of the Al proteins were conducted with the purified proteins in an end-point measurement. The amount of L-ribulose, product of Al activity with L-arabinose as a substrate, was quantified by HPLC (Dionex Ulimate 3000, with RI detector Shodex RI-101) using a Biorad Aminex HPX-87P column (with respective precolumn). The settings are the following:
eluent: MilliQ water
flow rate: 0.6 ml/min
column oven 80°C
run time: 40 min
injection volume 20 µl
detection: RI, 50 °C, Polarity (plus), Data Collection Rate 10 Hz

The calibration range for L-arabinose and L-ribulose are 0.25 - 10 mg/ml and 0.05 - 2 mg/ml, respectively.

Tests were conducted beforehand to establish the reaction conditions, esp. the amount of protein and reaction time to ensure a measurement in the linear range. The final composition of the reactions is shown in table 1.

| Component | Final concentration |
|---|---|
| HEPES pH 7 | 100 mM |
| MnCl₂ / MgCl₂ / CaCl₂ | 1/5 mM |
| L-arabinose | 400 mM |
| purified Al protein | 0.375 µM |

100 µl of a protein solution (purified Al diluted to 0,75 µM in 100 mM HEPES, pH 7) were added to 20 µl of a MnCl₂ / MgCl₂ / CaCl₂ solution (10 or 50 mM in 100 mM HEPES, pH 7). 80 µl of a concentrated L-arabinose solution (1 M in 100 mM HEPES, pH 7) were added and the reaction was mixed and centrifuged. The reactions were performed at 30 °C or 50 °C for 20 minutes in a thermocycler. The reactions were stopped by heat treatment at 95 °C for 10 min, then diluted 1:5 with water, filtered and analyzed by HPLC. Each reaction was done in triplicate. The enzyme activity was calculated from the amount of L-ribulose (in mole) produced after 1200 seconds by 75 picomoles of enzyme. The unit is kat/mol_{enzyme} with kat being mol_{ribulose}/s.

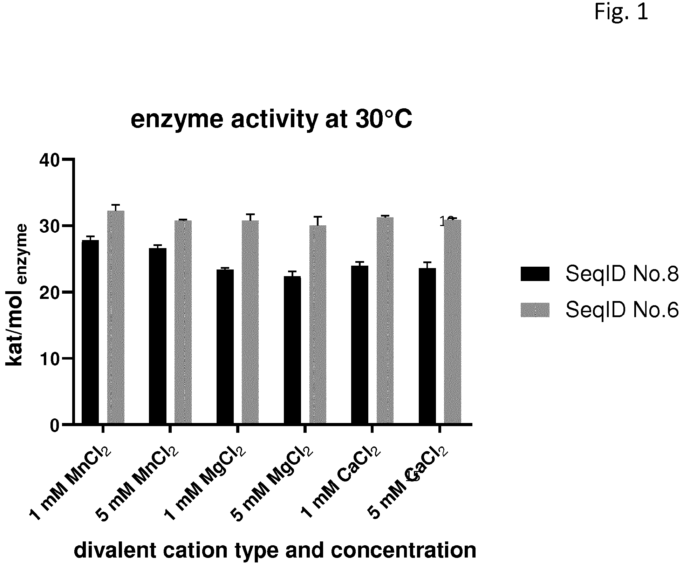

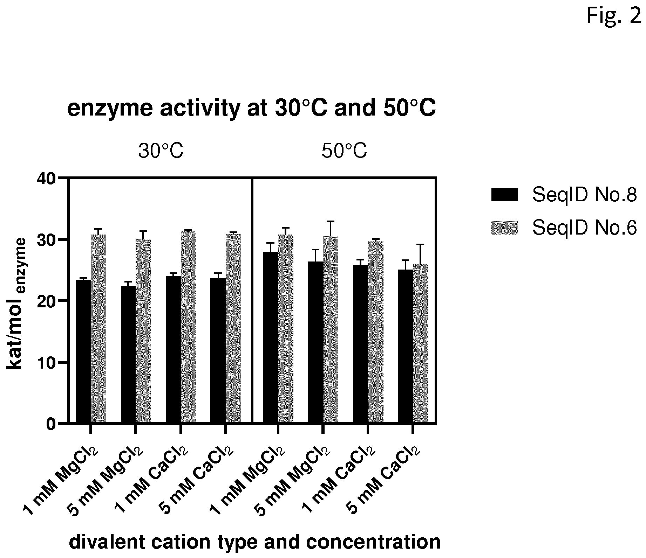

In figure 1 it can be seen, that at 30 °C the enzyme according to SEQ ID NO: 6 has a higher catalytic activity than the enzyme according to SEQ ID NO: 8 at low and high concentrations of all divalent metal cations. The catalytic activity is at least 15 % higher in the presence of Mn²⁺ and at least 30% higher in the presence of Mg²⁺ and Ca²⁺. Figure 2 shows furthermore that the catalytic activity of the enzyme according to SEQ ID NO: 6 is superior to the enzyme according to SEQ ID NO: 8 for divalent cations Mg²⁺ and Ca²⁺ at 30 °C and an elevated temperature of 50 °C. Taken together, this shows that the L-arabinose isomerase according to SEQ ID NO: 6 shows a significantly increased temperature tolerance compare to the enzyme according to SEQ ID NO: 8 with a high catalytic activity in the presence of high concentrations of divalent metal cations.

### Sequence Listing Description:

SEQ ID NO: 1: DNA sequence of modified L-arabinose isomerase, codon-optimized
SEQ ID NO: 2: amino acid sequence of modified L-arabinose isomerase
SEQ ID NO: 3: DNA sequence of wildtype L-arabinose isomerase, codon-optimized
SEQ ID NO: 4: amino acid sequence of wildtype L-arabinose isomerase from *Lactobacillus antri* DSM 16041
SEQ ID NO: 5: DNA sequence of modified L-arabinose isomerase, N-terminal 6xhis-tag, codon-optimized
SEQ ID NO: 6: amino acid sequence of modified L-arabinose isomerase, N-terminal 6xhis-tag
SEQ ID NO: 7: DNA sequence of wildtype L-arabinose isomerase, N-terminal 6xhis-tag, codon-optimized
SEQ ID NO: 8: amino acid sequence of wildtype L-arabinose isomerase, N-terminal 6xhis-tag
SEQ ID NO: 9: DNA sequence of modified L-arabinose isomerase, codon-optimized
SEQ ID NO: 10: DNA sequence of modified L-arabinose isomerase, codon-optimized
SEQ ID NO: 11: amino acid sequence of modified L-arabinose isomerase
SEQ ID NO: 12: DNA sequence of modified L-arabinose isomerase, codon-optimized
SEQ ID NO: 13: amino acid sequence of modified L-arabinose isomerase
SEQ ID NO: 14: DNA sequence of modified L-arabinose isomerase, N-terminal 6xhis-tag, codon-optimized
SEQ ID NO: 15: amino acid sequence of modified L-arabinose isomerase, N-terminal 6xhis-tag
SEQ ID NO: 16: DNA sequence of modified L-arabinose isomerase, N-terminal 6xhis-tag, codon-optimized
SEQ ID NO: 17: amino acid sequence of modified L-arabinose isomerase, N-terminal 6xhis-tag

## Claims

1. Temperature optimized L-arabinose isomerase with a high catalytic activity within feedstocks comprising a high concentration of divalent metal ions, having a sequence identity of at least 97.0 % to SEQ ID NO:2.

2. Temperature optimized L-arabinose isomerase according to claim 1 comprising SEQ. ID NO: 4 with at least one mutation selected from the group consisting of deletions and mutations at position 40, 41, 42, 43, 239, 240, 241, 242, 243, 244, 246, 247, 248, 249, 250 and 251.

3. Temperature optimized L-arabinose isomerase according to claim 2, wherein the at least one mutation is a substitution selected from the group consisting of substitutions at position 40, 41, 43, 239, 246, 247, 248, 249, 250 and 251.

4. Temperature optimized L-arabinose isomerase according to claim 2 or 3, wherein the at least one mutation is a substitution selected from the group consisting of A40K, S41K, K43D, M239I, H246P, D247K, K248A, Y249S, V250M and H251D.

5. Temperature optimized L-arabinose isomerase according to claim 2, wherein the at least one mutation is a deletion selected from the group consisting of deletions at position 42, 240, 241, 242, 243 and 244.

6. Temperature optimized L-arabinose isomerase according to claim 5, wherein the at least one mutation is a deletion selected from the group consisting of G42, V240, E241, G242, D243 and N244.

7. Temperature optimized L-arabinose isomerase according to claim 2, comprising SEQ. ID NO: 4 with a substitution at position 40, 41 and 43 and/or at position 239, 246, 247, 248, 249, 250 and 251.

8. Temperature optimized L-arabinose isomerase according to claim 2 or 7, comprising SEQ ID NO: 4 with a deletion at position 42 and/or at position 240, 241, 242, 243 and 244.

9. Temperature optimized L-arabinose isomerase according to any of the foregoing claims, having a sequence identity of at least 98.0 % to SEQ ID NO: 2.

10. Temperature optimized L-arabinose isomerase according to any of the foregoing claims, having a sequence identity of at least 99.0 % to SEQ. ID NO: 2.

11. Temperature optimized L-arabinose isomerase according to any of the foregoing claims selected from the group consisting of SEQ ID NO: 6, SEQ ID NO: 8, SEQ ID NO: 11, SEQ ID NO: 13, SEQ ID NO: 15 and SEQ ID NO 17.

12. Temperature optimized L-arabinose isomerase according to any of the foregoing claims, having a catalytic activity of at least 28.5 kat/mol enzyme within feedstocks of an L-arabinose content of 400 mM and a content of MgCl₂ of 5 mM when incubated at 30 °C for 20 minutes at pH 7.

13. Nucleic acid sequence encoding a temperature optimized L-arabinose isomerase according to any of claims 1 to 12.

14. Use of the temperature optimized L-arabinose isomerase according to any of claims 1 to 12 within a fermentation process of lignocellulosic hydrolysate.

15. Composition containing the temperature optimized L-arabinose isomerase according to any of claims 1 to 12.

16. Use of the composition according to claim 15 within a fermentation process of lignocellulosic hydrolysate.

17. Vector comprising the nucleic acid sequence according to claim 13.

18. Yeast cell comprising the nucleic acid sequence according to claim 13.

19. Use of the yeast cell according to any of claims 18 within a fermentation process of feedstock with a high content of divalent metal ions.
